(19)

**Europäisches Patentamt**
**European Patent Office**
**Office européen des brevets**

(11) **EP 1 725 876 B1**

(12) **EUROPEAN PATENT SPECIFICATION**

(45) Date of publication and mention
of the grant of the patent:
**06.05.2009 Bulletin 2009/19**

(21) Application number: **05717315.5**

(22) Date of filing: **03.03.2005**

(51) Int Cl.:
*G01N 33/74* (2006.01)     *G01N 33/573* (2006.01)

(86) International application number:
**PCT/FI2005/050061**

(87) International publication number:
**WO 2005/085871 (15.09.2005 Gazette 2005/37)**

(54) **A METHOD FOR PREDICTING THE STATE OF THE GASTRIC MUCOSA**

VERFAHREN ZUR VORHERSAGE DES ZUSTANDS DER MAGENSCHLEIMHAUT

PROCEDE PERMETTANT DE PREDIRE L'ETAT DE LA MUQUEUSE GASTRIQUE

(84) Designated Contracting States:
**AT BE BG CH CY CZ DE DK EE ES FI FR GB GR HU IE IS IT LI LT LU MC NL PL PT RO SE SI SK TR**

(30) Priority: **05.03.2004 FI 20040359**

(43) Date of publication of application:
**29.11.2006 Bulletin 2006/48**

(73) Proprietor: **BIOHIT OYJ**
**00880 Helsinki (FI)**

(72) Inventors:
• **SARNA, Seppo**
**FI-02170 Espoo (FI)**
• **TIUSANEN, Tapani**
**FI-01280 Vantaa (FI)**
• **SUOVANIEMI, Osmo**
**FI-00570 Helsinki (FI)**

(74) Representative: **Matilainen, Mirja Helena et al**
**Oy Jalo Ant-Wuorinen AB,**
**Iso Roobertinkatu 4-6 A**
**00120 Helsinki (FI)**

(56) References cited:
EP-A2- 0 760 482        WO-A1-00/67035
WO-A1-02/054084        WO-A1-03/029826
WO-A1-20/04023148        WO-A2-01/77685
US-B1- 6 861 510        US-B2- 6 696 262

• **PASECHNIKOV V. ET AL:** 'Invasive and non-invasive diagnosis of Helicobacter pylori-associated atrophic gastritis: A comparative study.' SCANDINAVIAN JOURNAL OF GASTROENTEROLOGY vol. 40, 2005, pages 297 - 301, XP002988879
• **PASECHNIKOV D. ET AL:** 'Possibility of non-invasive diagnose of gastric mucosal precancerous changes.' WORLD J GASTROENTEROL vol. 10, no. 21, 2004, pages 3146 - 3150, XP002988880
• **PASECHNIKOV V. ET AL:** 'The non-invasive diagnosis of precancerous changes of stomach mucosa.' ANNALES ACADEMIAE MEDICAE BIALOSTOCENSIS. vol. 49, 2004, pages 66 - 71, XP002988881
• **VOONONEN H. ET AL:** 'Non-endoscopic diagnosis of atrophic gastritis with a blood test. Correlation between gastric histology and serum levels of gastrin-17 and pepsinogen I: a multicentre study.' EUROPEAN JOURNAL OF GASTROENTEROLOGY & HEPATOLOGY. vol. 15, no. 8, 2003, pages 885 - 891, XP002988882
• **HALLISSEY M. ET AL:** 'Evaluation of Pepsinogen A and Gastrin-17 as Markers of Gastric Cancer and High-Risk Pathologic Conditions.' SCAND.J.GASTROENTEROL vol. 29, 1994, pages 1129 - 1134, XP002988883
• **SIPPONEN P. ET AL:** 'Serum Levels of Amidated Gastrin-17 and Pepsinogen I in Atrophic Gastritis: An Abservational Case-Control Study.' SCAND J GASTROENTEROL vol. 37, 2002, pages 785 - 791, XP002988884

**Description**

FIELD OF THE INVENTION

The present invention is directed to a method for assessing or predicting the state or condition of the gastric mucosa, by determining the probability for the gastric mucosa of a subject belonging to at least one gastric mucosa class or category. In the method, the concentration of specific mucosa specific analytes, such as the pepsinogen I concentration, the gastrin-17 concentration as well as the concentration or presence of a *Helicobacter pylori* marker, is determined in the subject, and data processing means are used to determine the probability for the gastric mucosa of the subject belonging to the at least one gastric mucosa class or category.

BACKGROUND OF THE INVENTION

Although the occurrence of new cases of gastric cancer has diminished in the recent years, gastric cancer is still one of the most common malignancies. In Finland, appr. 250 to 300 new cases of cancer/one million people/year are registered. In the age group of people above 50, there are an estimated 2350 cases of stomach cancer, which is about 3 per mill of the age group population (Finnish Cancer Registry - The Institute for Statistical and Epidemiological Cancer Research 1993). In addition to Finland, there is a high gastric cancer incidence in Iceland, South America and especially in Japan and China.

The prognosis of gastric cancer is usually poor, as there is no specific treatment. Presently the only possibility of successfully treating gastric cancer is its early detection and total removal surgically.

Gastric cancer does not necessarily give any symptoms in its early stages. The late appearance of symptoms naturally delays the patient from seeking treatment. On the other hand, the clinical findings in the early stage of gastric cancer are often non-specific. The primary diagnostic method for gastric cancer is presently gastroscopy and biopsies, cell and aspiration cytology associated therewith. As routine gastroscopies are carried out in order to examine symptoms, such as pain in the upper abdomen or bleeding of the gastrointestinal tract, a symptomatic gastric cancer discovered in this manner is often already far advanced and thus inoperable. Attempts have also been made at improving primary diagnostics with various immunological methods, but no sufficiently specific immunological method has been successfully developed.

It is a primary object to find the means by which it would be possible to identify within the general population easily and with moderate costs those persons which might be suffering from gastric cancer in its initial stages. After identification these persons should immediately be examined by gastroscopy. At the same time those persons could be identified which exhibit premalignant gastric changes which need to be followed up.

Gastric cancer can be preceded by a number of different gastric diseases or conditions (so called precancerous conditions), which are chronic atrophic gastritis, pernicious anaemia, ventricular ulcer, gastric polyposis and the Ménétrier disease (giant hypertrophic gastritis). Clearly identifiable changes of the mucosa are dysplasia and adenoma. The said conditions are associated with an appr. 4 to 5 fold relative cancer risk, as compared to the general population. It has been established that in almost all diseases the risk is mediated over chronic atrophic gastritis.

Chronic gastritis means a prolonged inflammatory condition of the gastric mucosa. The disease can coarsely be divided into the so-called superficial and the atrophic form. In superficial gastritis, the inflammatory cell infiltration is concentrated below the surface epithelium. In case the inflammation progresses and diffuses between the specific gastric secretory glands, one refers to chronic atrophic gastritis. In such a case, the normal glandular structures of the gastric mucosa are at least partly substituted by metaplastic changes.

The relative risk of gastric cancer in patients suffering from atrophic gastritis in the corpus area of the stomach, has been estimated, as calculated from the Finnish cancer statistics, to be about 4- to 5-fold as compared to persons having a healthy mucosa. In addition, there is a risk for falling ill with pernicious anaemia due to intrinsic factor deficiency and B12 vitamin absorption disturbance. In severe atrophy of the antrum area, the risk is even 18-fold. If atrophic changes appear both in the antrum and the corpus area (pangastritis), the risk can increase to even 90-fold (Sipponen, P, Kekki, M, Haapakoski, J. Ihamäki, T & Siurala, M (1985) Gastric cancer risk in chronic atrophic gastritis: statistical calculations of cross-sectional data. Int J Cancer 35:173-77).

*Helicobacter pylori* is a spiral shaped, gram-negative bacterium which thrives in the mucus in the immediate vicinity of the surface epithelial cells of the gastric mucosa and in the cell interstices. The bacterium apparently is transmitted perorally from one person to the other. The effect of the bacterium on the gastric mucosa is an inflammation reaction, which is mediated over a complement by liberating strong inflammation mediator substances. After the acute stage, the inflammation is transformed into chronic gastritis. In patients suffering from chronic gastritis, in 70 to 90 % a *Helicobacter pylori* infection can be established (Calam, J (1994) Helicobacter pylori (Review) Eur. J. Clin Invest 24: 501-510). As *Helicobacter pylori* infection and chronic gastritis in the stomach are closely associated, it has been stipulated that this bacterial infection could be one etiological factor in the development of stomach cancer. It is for this reason possible that eradication of the *Helicobater pylori* bacteria in the initial stages of the infection, could prevent the development of atrophy associated with chronic gastritis, and thus reduce the cancer risk and the risk of peptic ulcers.

The publication WO 96/15456 discloses a method for screening for the risk of cancer by determining the concentration of the analytes pepsinogen I, and gastrin-17 from a serum sample of a subject. According to the said publication, the so determined concentration values are then compared to a cut-off value and a reference value for each analyte. A serum pepsinogen I concentration below the cut-off value for pepsinogen I in combination with a gastrin-17 concentration value above the upper reference limit indicates severe atrophy of the corpus area of the stomach. A serum gastrin-17 level below the cut-off value for gastrin-17 in combination with a pepsinogen I value above the cut-off value for pepsinogen I on the other hand indicates atrophy of the antrum area of the stomach. In case the serum pepsinogen I is below the cut-off value for pepsinogen I, and the gastrin-17 level is at the lower limit of its reference value, this is an indication of severe atrophy in the whole stomach, *i.e.* of atrophic pangastritis. According to an embodiment disclosed, the said tests may be combined with a test for *Helicobacter pylori* antibodies.

According to the said WO-publication, the method can be supplemented with a so-called protein stimulation test, according to which a blood sample is taken in the morning after fasting, whereafter the patient eats a protein-rich standard meal and blood samples are taken at 15 minute intervals for two hours. The maximal increase is evident after appr. 20 minutes. In case the atrophy is located in the antrum, there will be a strongly reduced response in this test. When the atrophy is located in the corpus, the response will be normal of increased, whereas atrophy of the whole mucosa leads to a reduced response.

The WO-publication WO 00/67035 discloses a method for assessing the risk of peptic ulcer by determining quantitatively the concentration of serum pepsinogen I and serum gastrin-17. According to this method, if both the measured serum pepsinogen I and gastrin-17 values are high, above the upper limit of their respective reference values, or the serum pepsinogen I value is above the upper limit of its reference value in combination with a gastrin-17 value within the reference range or below its cut-off value, this is an indication of an increased risk of peptic ulcer.

The publication WO 02/054084 discloses a method for assessing the condition of the gastric mucosa, by determining the concentration of pepsinogen I, gastrin-17 and a marker for *Helicobacter pylori* infection and comparing the same to the cut off values for the said markers, to obtain, using data processing means, a combination of comparison results specific for the subject tested. The publication WO 2004/023148 discloses a method for detecting a risk of gastric acid related disease, such as Barrett's esophagus, by determining the concentration of pepsinogen I, fasting gastrin-17 and a marker for *Helicobacter pylori* infection and comparing the same to the cut off values for the said markers.

Methods are known in the art for measuring the concentrations of the various analytes, and there are also commercially available kits for this purpose. Some exemplatory methods for carrying out the said determinations are described in the WO-publication 96/15456 as well.

SUMMARY OF THE INVENTION

The object of the invention is a method for assessing or predicting the state of the gastric mucosa in a subject by determining, in said subject, the probability for the gastric mucosa belonging to at least one gastric mucosa class, the method comprising

- measuring, from a sample of said subject, the pepsinogen I (PGI) and gastric-17 (G-17) analyte concentrations, as well as determining the presence or concentration of a marker for *Helicobacter pylori,*
- entering the data so obtained in a data processing system comprising an operating system, a database and means for transceiving and processing data, the said data processing system being adapted to determine the probability for the gastric mucosa belonging to the at least one gastric mucosa class, based on the data entered as well as on predefined clinical data in the database, the predefined clinical data in the database comprising data obtained from a reference population group by gastroscopic study and determination of the PGI and G-17 analytes and *Helicobacter pylori* marker in said reference population group, the information so generated by the data processing system being indicative of the state of the gastric mucosa in said subject.

According to the invention the generated information can thus be used for the assessment or the prediction of the state of the gastric mucosa in said subject.

The invention is also directed to a kit and to a computer program product especially for use in the method according to the invention.

The kit according to the invention comprises means for determining, from a sample, the pepsinogen I and gastrin-17 concentration, and the concentration or presence of a *Helicobacter pylori* marker, as well as a computer program product embodied on a computer readable medium and comprising computer code means adapted to determine a probability for a gastric mucosa class, based on measured values for said analytes and/or marker, as well as predefined clinical data in a data base, the predefined clinical data in the database comprising data obtained from a reference population group by gastroscopic study and determination of the PGI and G-17 analytes and *Helicobacter pylori* marker in said reference population group, and to provide information in response to said determination and optimally to other entered data, when run on a computer.

The computer program product according to the invention is embodied on a computer readable medium and comprises computer code means adapted to determine a probability for a gastric mucosa class, based on measured values for the PGI and G-17 analytes and *Helicobacter pylori* marker, as well as predefined clinical data in a database and to provide information in response to said determination and optionally to other entered data, when run on a computer.

DETAILED DESCRIPTION OF THE INVENTION

In this invention, the term "probability for a gastric mucosa class" means the probability for the gastric mucosa of the subject to be tested to belong in a gastric mucosa class.

The term "gastric mucosa class" of a subject refers to the gastric mucosa of a subject being classified as being normal (N), exhibiting superficial or non-atrophic gastritis (S), corpus atrophy (C), antrum atrophy (A), or antrum + corpus atrophy (AC), respectively. The invention makes it possible to determine the probability of the subjects stomach of belonging to one or more of the said classes, and/or to determine the probability distribution of a number of classes.

In the present invention, the clinical database comprises data obtained from gastroscopic studies and biopsy, corresponding concentrations for mucosa specific analytes and/or markers, optionally other data entered, and information on different classes to predict. The probabilities are preferably determined using a statistical method, and the preferred statistical method for calculating classification probabilities is the multinominal logistic regression method. The predefined clinical data in the database comprises data obtained from a reference population group by gastroscopic study and determination of the PGI and G-17 analytes and *Helicobacter pylori* marker in said reference population group.

According to the present invention the *Helicobacter pylori* marker is a *Helicobacter pylori* antibody, the concentration of which is measured from a sample or the *Helicobacter pylori* antigen, the presence of which is determined in the sample. The gastrin value that is measured is the stimulated gastrin-17 value (G-17st), or both the fasting (basal) gastrin-17 and the stimulated gastrin-17.

In one embodiment of the invention, in addition, the concentration of the analyte pepsinogen II (PGII) is measured, and the ratio PGI/PGII is used in the statistical calculation.

According to the invention the analytes are measured from a body fluid, such as a serum whole blood, urine, saliva or lacrimal fluid sample, especially a serum sample.

According to one embodiment of the invention, the generated information advantageously relates to at least one gastric mucosa class probability, whereby an estimate of the change in the said probability can be used to provide information as to the change in the state of the gastric mucosa.

The data processing means can comprise a display on which the generated information is displayed.

The basic statistical method used in the invention to classify the gastric mucosa is based on stochastic multi nominal logistic regression analysis (MLR) run on clinical data. The benefit of a stochastic version compared to a simpler deterministic approach, where a value is rated based on a simple cut-off analysis, is that it relies on the fact that stochastic models consider uncertainty as an important aspect of the problem. This means that the stochastic approach is less insensitive to different kinds of errors and random variation, which do exist in a complex process like this and is clearly the preferred method to use.

Logistic regression is preferred over linear because it solves the problems related to the "classical regression assumptions" i.e. heteroskedasticity of error terms, normal distribution requirement and negative (or >1) probabilities. This means that the logistic regression is often preferred because it does not require a linear relationship between the dependent and independent variables, which is the case with most clinical assays, where the relationship typically follows an S-shaped curve.

Multinominal logistic regression is used where there are several dependent variables, in the case of the invention gastric mucosa classes. A general multi nominal logistic regression model is presented as follows:

$$\ln (P / 1 - P) = BX + E,$$

wherein,

P is a vector (n x 1) containing the response variable (class probability)

B is a vector (q x 1) containing the model parameters

X is a matrix (n x q) containing the q factors

E is a vector (n x 1) containing the noise terms

wherein n is the number of classes and q is the number of parameters.

Prior to creating the models the values for the coefficients $B_j$ are calculated using the maximum likelihood estimates method of the different classes from clinical data in a database.

As the different classes of gastric mucosa are predefined, each combination of independent variables generates different coefficients of $B_j$. Therefore, a model based method for estimating probabilities requires several models covering all the possible combinations of entered data. If the combination is limited, and if the database is statistically big enough, this is the preferred method of choice due to its computational simplicity.

If the database is statistically small and there is an obviously clear unbalance between the data available for the different classes, a combination of parameters may gain better estimates than the others. Two variants of the possible modification of the model-based method are presented here. An "iterative method" uses all the parameters entered for the first probability estimates. Depending on the outcome, it automatically uses a subset of entered parameters for new calculation of probability estimates. A "permutative method", on the other hand, uses all the different model permutations to calculate probability estimates to find out the most probable class for the entered parameters.

If the database is statistically small or dynamic in nature and the data processing system is fast enough, the probability estimates can be calculated using the maximum likelihood estimates on the entered data in real time.

Either the model parameters or especially the database can be located at a distance from the data processing system calculating the probabilities for the entered data.

In case of a remote database it is practical to set up a database server with a server based application re-running the models every time new clinical data is entered to the clinical database. The remote client application where parameters are entered fetches the model parameters from the remote database server for probability calculations. It is obvious that Internet technology provides the information highway for the data transmission between the client and the server systems.

According to the invention, the continuous probability value as a parameter provides means for detecting a change of state of classes in question. By resampling the patient at a later time, information on probability development with time enables better diagnosis or a suggestion for treatments or further investigations and/or tests based on the results so obtained.

The clinical data is collected by assaying patients and by classifying patients stomachs in different groups, whereby the gastric mucosa classes N = normal antrum, S = superficial, C = Corpus atrophy, A = Antrum atrophy, AC = Antrum + Corpus atrophy are obtained, based on gastroscopy and biopsy studies.

A statistical run is then performed to find out constants for the different models. A model based application program uses the predefined models to calculate probabilities for the different classes (N, S, C, A, AC) on the parameters entered.

If the result indicates a non-normal case with a present helicobacter infection, treatment is started. After a period of time (e.g. 3 months) a new assaying can be performed. By comparing the probability distribution to the earlier ones, a treatment result can be predicted.

The suggested diagnosis provided is based on the maximum likelihood estimates of the most probable gastric mucosa class on the measured assay levels. The diagnosis of the most probable case displayed is hardly ever 100% probable, but rather much lower, but still higher than any other option. In fact the stochastic version displays these probabilities to every gastric mucosa class to view the difference. It can then easily be seen what the probability is for healthy vs. abnormal values. The probability distribution provides important information for doctors not only in suggesting a diagnosis but also in guiding for re-test or further investigations.

As the presented stochastic method provides for the most probable gastric mucosa class, it offers one more benefit over a deterministic model. As soon as treatment e.g. eradication therapy for *Helicobacter pylori* has been carried out, a blood sample can easily be taken 3 to 6 months later and simple and non-expensive serological assays be carried out. By following the assay levels, and especially the predicted probabilities, a possible healing can be detected by simply plotting the probabilities vs. time or by using a mathematical method on time series to predict a change of state.

In one preferred embodiment of the invention the maximum likelihood estimate calculations for classification probabilities based on entered analyte or marker data can be done at the same time on the existing database or by using pre-calculated multinominal regression models run earlier on a clinical database.

According to one embodiment of the invention a series of measurements with classification probabilities are combined which enables that information of a change, e.g. healing of the stomach mucosa, can be predicted.

In assessing the change in the state of the gastric mucosa over time, the probability for the at least one gastric mucosa class is re-determined, at a later point of time, and the probabilities so calculated are compared in order to estimate a change in the said probabilities and to provide information relating to the change in the state of the gastric mucosa.

The generated information is advantageously used to generate a diagnosis or a suggestion for treatment or further investigations and/or tests.

The following examples are intended to illustrate the invention without restricting it in any way. The analytes measured are pepsinogen I, gastrin-17 and *Helicobacter pylori* antibodies. The probabilities for each gastric mucosa category are determined by multinominal logistic regression. Also the respective diagnosis is given in each example, together with further suggestions for examinations and treatments, when applicable.

EXAMPLE 1

The following analyte concentrations were measured from a sample of a patient.

| | | |
|---|---|---|
| Pepsinogen I | 7 | µg/l |
| Gastrin-17 (post pr.) | 49.9 | pmol/l |
| *H. pylori* IgG: | 156 | EIU |

Based on the concentrations determined, the following probabilities were calculated.

| Probabilities: | | |
|---|---|---|
| NORMAL | 0.2 | % |
| ANTRUM ATROPHY | 0 | % |
| ANTRUM AND CORPUS ATROPHY | 19 | % |
| CORPUS ATROPHY | 66.7 | % |
| NON-ATROPHIC GASTRITIS | 13.8 | % |

Based on the calculated probabilities a diagnosis of atrophic corpus gastritis is suggested. Such a diagnosis is associated with:

1. Increased risk of gastric cancer (risk factor 5X).
2. Peptic ulcer disease (duodenal or gastric) is unlikely.
3. *Helicobacter pylori* infection.

EXAMPLE 2

The following analyte concentrations were measured from a sample of a patient

| | | |
|---|---|---|
| Pepsinogen I | 87 | µg/l |
| Gastrin-17 | 0.3 | pmol/l |
| *H. pylori* IgG: | 12 | EIU |

Based on the concentrations determined, the following probabilities were calculated.

| Probabilities: | | |
|---|---|---|
| NORMAL | 84.5 | % |
| ANTRUM ATROPHY | 6.2 | % |
| ANTRUM AND CORPUS ATROPHY | 0 | % |
| CORPUS ATROPHY | 0.1 | % |
| NON-ATROPHIC GASTRITIS | 8.9 | % |

Based on the calculated probabilities a diagnosis of a normal mucosa is suggested. Such a diagnosis is associated with:

1. Very low risk of gastric cancer.
2. Very low risk of peptic ulcer.
3. No *Helicobacter pylori* infection.

EXAMPLE 3

The following analyte concentrations were measured from a sample of a patient

| | | |
|---|---|---|
| Pepsinogen I | 7 | μg/l |
| Pepsinogen II | 3 | μg/l (PGI/PGII: 2.3) |
| Gastrin-17 | 20 | pmol/l |
| *H. pylori* IgG: | 48 | EIU |

Based on the concentrations determined, the following probabilities were calculated.

| Probabilities: | | |
|---|---|---|
| NORMAL | 2.6 | % |
| ANTRUM ATROPHY | 1.9 | % |
| ANTRUM AND CORPUS ATROPHY | 16.1 | % |
| CORPUS ATROPHY | 73.5 | % |
| NON-ATROPHIC GASTRITIS | 5.6 | % |

Based on the calculated probabilities a diagnosis of atrophic corpus gastritis is suggested. Such a diagnosis is associated with:

1. Increased risk of gastric cancer (risk factor 5X).
2. Peptic ulcer disease (duodenal or gastric) is unlikely.
3. *Helicobacter pylori* infection.

EXAMPLE 4

The following analyte concentrations were measured from a sample of a patient

| | | |
|---|---|---|
| Pepsinogen I | 11.5 | μg/l |
| Pepsinogen II | 1.6 | μg/l (PGI/PGII: 7.1) |
| Gastrin-17 | 33.1 | pmol/l |
| *H. pylori* IgG: | 26.9 | EIU |

Based on the concentrations determined, the following probabilities were calculated.

| Probabilities: | | |
|---|---|---|
| NORMAL | 25.6 | % |
| ANTRUM ATROPHY | 6.9 | % |
| ANTRUM AND CORPUS ATROPHY | 5.5 | % |
| CORPUS ATROPHY | 49.3 | % |
| NON-ATROPHIC GASTRITIS | 12.4 | % |

Based on the calculated probabilities a diagnosis of atrophic corpus gastritis is suggested. Such a diagnosis is associated with:

1. Increased risk of gastric cancer (risk factor 5X).
2. Peptic ulcer disease (duodenal or gastric) is unlikely.
3. No *Helicobacter pylori* inflection - atrophic gastritis probably autoimmune in origin.

EXAMPLE 5

The following analyte concentrations were measured from a sample of a patient.

| Pepsinogen I | 14.2 | $\mu$g/l |
| Pepsinogen II | 1.7 | $\mu$g/l (PGI/PGII: 8.3) |
| Gastrin-17 | 21.7 | pmol/l |
| *H. pylori* IgG: | 27 | EIU |

Based on the concentrations determined, the following probabilities were calculated.

| Probabilities: | | |
| NORMAL | 38.2 | % |
| ANTRUM ATROPHY | 7.5 | % |
| ANTRUM AND CORPUS ATROPHY | 5.7 | % |
| CORPUS ATROPHY | 33.1 | % |
| NON-ATROPHIC GASTRITIS | 15.2 | % |

Based on the calculated probabilities a diagnosis of a normal mucosa is suggested. Such a diagnosis is associated with:

1. Very low risk of gastric cancer.
2. Very low risk of peptic ulcer.
3. No *Helicobacter pylori* infection.

EXAMPLE 6

The following analyte concentrations were measured from a sample of a patient

| Pepsinogen I | 14.7 | $\mu$g/l |
| Pepsinogen II | 2.2 | $\mu$g/l (PGI/PGII: 6.6) |
| Gastrin-17 | 24.2 | pmol/l |
| *H. pylori* IgG: | 25.7 | EIU |

Based on the concentrations determined, the following probabilities were calculated.

| Probabilities: | | |
| NORMAL | 30.6 | % |
| ANTRUM ATROPHY | 6.2 | % |
| ANTRUM AND CORPUS ATROPHY | 6.3 | % |
| CORPUS ATROPHY | 42.9 | % |
| NON-ATROPHIC GASTRITIS | 13.9 | % |

Based on the calculated probabilities a diagnosis of atrophic corpus gastritis is suggested. Such a diagnosis is associated with:

1. Increased risk of gastric cancer (risk factor 5X).
2. Peptic ulcer disease (duodenal or gastric) is unlikely.
3. No *Helicobacter pylori* infection - atrophic gastritis probably autoimmune in origin.

The agreement with the reference method, i.e. gastroscopy and biopsy, is dependent on the gastric mucosa class and the model used. The overall agreement using a model with PGI, PGII, G17st and HPab is 80.2%. For class N, a normal healthy mucosa, the overall agreement is 90.8%. For corpus atrophy (C) and non-atrophic gastritis (S) the result is 85.5% and 74.0% respectively. The corresponding values for classes A and AC are somewhat lower, as these classes are more difficult to predict.
It has also been shown in tests that the highest class so predicted corresponds to the same gastritis class finding with gastroscopy with a much higher percentage. E.g. if the most probable class is predicted with a probability of 65%, the same class finding with gastroscopy is achieved in 90% of the cases. Therefore, if the most probable class is predicted with over 90% probability, there is practically a one-to-one correspondence with gastroscopy and biopsy.

BRIEF DESCRIPTION OF THE DRAWINGS

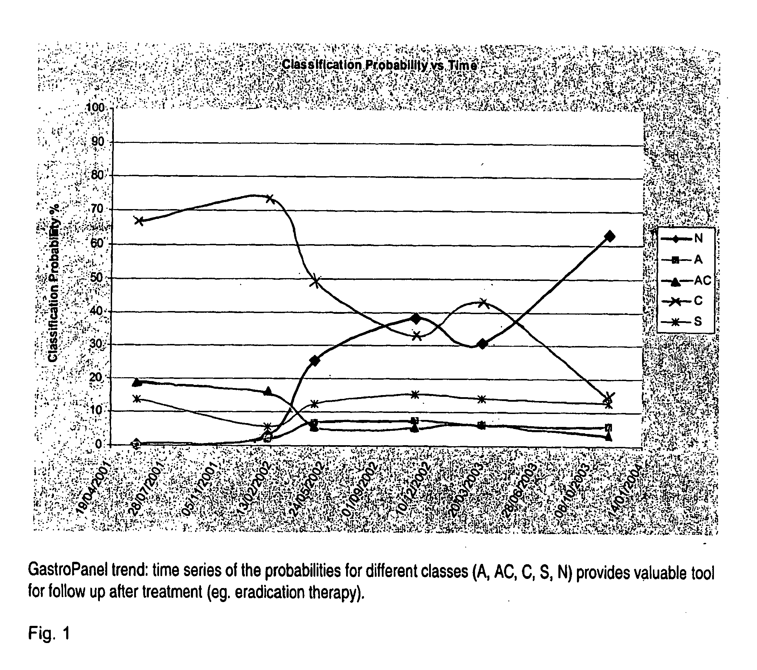

In fig. 1 the classification probability for the patient according to Example 1, whereby the first assay results for PGI and G17 relate to the values given in Example 1, as a function of time is presented for the different classes (N, A, AC, C, S). Five sets of assays were made during a period of two years. The patient was treated to eradicate *Helicobacter.* It can bee seen that when treatment is put in, the probability for corpus gastritis is decreasing, after 3 months the probability having decreased from 70% to 50% and after 8 months it is about 33%. Also the probability for corpus and antrum gastritis has decreased from 20% to around 5% during the same time. The probability for a normal healthy mucosa has in this time increased from almost 0% to almost 40%.

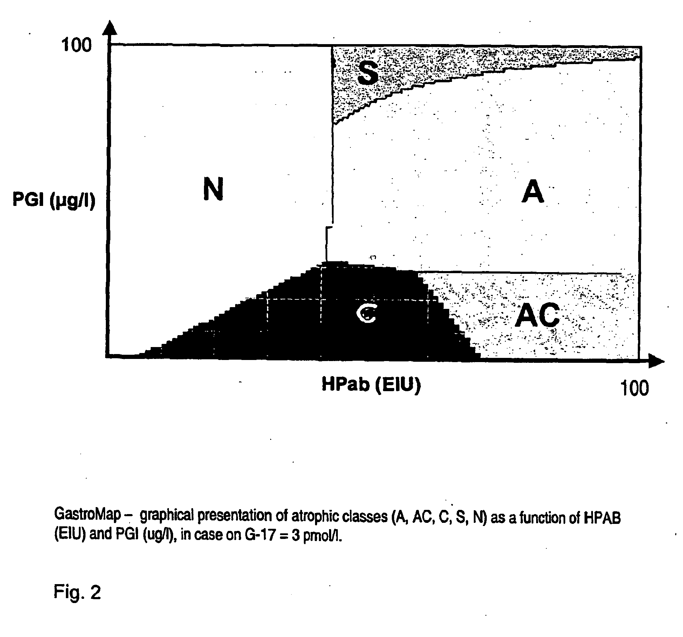

Fig 2 is a graphical illustration, a probability 'map', generated from the quantitative probability values for the different gastric mucosa classes. In case of three quantitative marker values, each point in the space represents the most probable class and its probability value. The present figure 2 displays a case where G-17 is set to 3 pmol/l, providing a two dimensional presentation of the map with PGI and HP-ab as the axes. From the figure, by inserting the measured values for PGI and HPab into the coordinate system, it can be seen which of the classes (A, AC, C, S, N) is the most probable. If the point is situated far from other class regions (A, AC, C, S, N), the probability for that class is high, if the point is situated close to the border to another region, the result is more uncertain, and another possible class need to be considered.

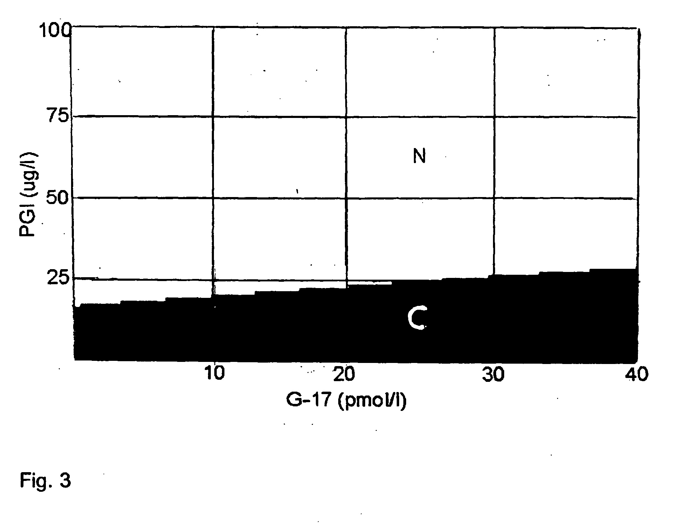

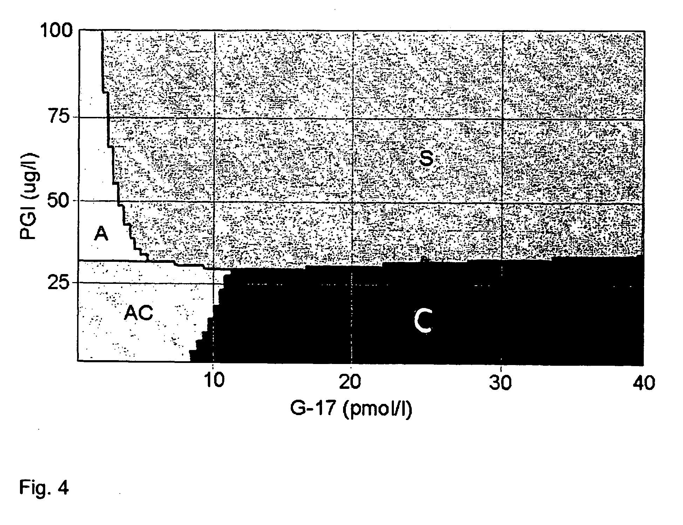

If the HP result (negative or positive) is known from serological or other tests, two maps, one for HP - (Fig. 3) and one for HP + (Fig. 4) provides a practical aid for classification. In this case the axes are obviously PGI and G-17. In general, by presenting the most probable classes on a map provide not only a practical way for document form classification, but a visual aid when run and displayed on a data processing display e.g. when samples from different patient groups are (e.g. no smokers - smokers, women - men, treated - non treated) are plotted on the same map. Such a visual aid provides easy means to compare groups with each other.

**Claims**

1.  Method for assessing or predicting the state of the gastric mucosa in a subject by determining, in said subject, the probability for the gastric mucosa belonging to at least one gastric mucosa class, the method comprising

    - measuring, from a sample of said subject, the pepsinogen I (PGI) and gas trin-17 (G-17) analyte concentrations, as well as determining the presence or concentration of a marker for *Helicobacter pylori,*
    - entering the data so obtained in a data processing system comprising an operating system, a database and means for transceiving and processing data, the said data processing system being adapted to determine the probability for the gastric mucosa belonging to the at least one gastric mucosa class, the gastric mucosa class being selected from the group of classes consisting of normal (N), antrum atrophy (A), antrum and corpus atrophy (AC), corpus atrophy (C) and superficial or non-atrophic gastritis (S), based on the data entered as well as on predefined clinical data in the database, the predefined clinical data in the database comprising data obtained from a reference population group by gastroscopic study and determination of the PGI and G-17 analytes and *Helicobacter pylori* marker in said reference population group, the information so generated by the data processing system being indicative of the state of the gastric mucosa in said subject.

2.  The method according to claim 1 for assessing a change in the state of the gastric mucosa, the method comprising repeating the determination of the probability for the at least one gastric mucosa class, and comparing the probabilities so obtained with the earlier determined probabilities in order to provide information relating to the change in the state of the gastric mucosa.

3.  The method according to any one of the preceding claims wherein the probabilities are determined using a statistical method for calculation of the classification probabilities.

4.  The method according to claim 3 wherein the statistical method for the calculation of the classification probabilities is a multinominal logistic regression method (MLR).

5.  The method according to any one of the preceding claims, comprising the further step of using the generated information for providing a diagnosis and/or a suggestion for further treatments or examinations.

6. The method according to any one of the preceding claims, wherein the *Helicobacter pylori* marker is a *Helicobacter pylori* antibody, the concentration of which is measured from the sample.

7. The method according to any one of the preceding claims, wherein the *Helicobacter pylori* marker is the *Helicobacter pylori* antigen, the presence of which is determined in the sample.

8. The method according to any one of the preceding claims, wherein the gastrin value measured is the stimulated gastrin-17 value (G-17st), or both the gastrin-17 and the stimulated gastrin-17.

9. The method according to any one of the preceding claims, wherein, in addition, the concentration of the analyte pepsinogen II (PGII) is measured, and the ratio PGI/PGII is used in the statistical calculation.

10. The method according to claim 1, wherein the analytes are measured from a body fluid, such as a serum whole blood, urine, saliva or lacrimal fluid sample, especially a serum sample.

11. The method according to any one of the preceding claims, wherein the data processing means comprise a display, and the information generated is displayed on the display.

12. A kit comprising means for determining, from a sample, the pepsinogen I and gastrin-17 concentration, and the concentration or presence of a *Helicobacter pylori* marker, as well as a computer program product embodied on a computer readable medium and comprising computer code means adapted to determine a probability for a gastric mucosa class, the gastric mucosa class being selected from the group of classes consisting of normal (N), antrum atrophy (A), antrum and corpus atrophy (AC), corpus atrophy (C) and superficial or non-atrophic gastritis (S), based on measured values for said analytes and/or marker as well as predefined clinical data in a database, the predefined clinical data comprising data obtained from a reference population group by gastroscopic studies and determination of values for PGI and G-17 analytes and *Helicobacter pylori* marker from said reference population group, and to provide information in response to said determination and optionally other entered data, when run on a computer.

13. A computer program product embodied on a computer readable medium and comprising computer code means adapted to determine a probability for a gastric mucosa class, the gastric mucosa class being selected from the group of classes consisting of normal (N), antrum atrophy (A), antrum and corpus atrophy (AC), corpus atrophy (C) and superficial or non-atrophic gastritis (S), based on measured values for the PGI and G-17 analytes and *Helicobacter pylori* marker, as well as predefined clinical data in a database, and to provide information in response to said determination and optionally to other entered data, when run on a computer.

**Patentansprüche**

1. Verfahren zum Beurteilen oder Voraussagen des Zustands der Magenschleimhaut in einem Patienten, indem in dem Patienten die Wahrscheinlichkeit bestimmt wird, dass die Magenschleimhaut mindestens einer Magenschleimhautklasse angehört, wobei das Verfahren Folgendes umfasst:

- Messen der Konzentrationen der Analyten Pepsinogen I (PGI) und Gastrin-17 (G-17) in einer Probe des Patienten sowie Bestimmen der Gegenwart oder Konzentration eines Markers für *Helicobacter pylori,*
- Eingeben der auf diese Weise erhaltenen Daten in ein Datenverarbeitungssystem, umfassend ein Betriebssystem, eine Datenbank sowie Mittel zum Übertragen und Verarbeiten von Daten, wobei das Datenverarbeitungssystem geeignet ist, die Wahrscheinlichkeit zu bestimmen, dass die Magenschleimhaut der mindestens einen Magenschleimhautklasse angehört, wobei die Magenschleimhautklasse unter der Gruppe von Klassen gewählt wird, die aus Folgendem besteht: normal (N), Antrumatrophie (A), Antrum- und Corpusatrophie (AC), Corpusatrophie (C) und oberflächliche oder nichtatrophische Gastritis (S), und zwar auf der Basis der eingegebenen Daten sowie auf der Basis vordefinierter klinischer Daten in der Datenbank, wobei die vordefinierten klinischen Daten in der Datenbank Daten umfassen, die in einer Referenzpopulation gewonnen wurden, und zwar durch gastroskopische Untersuchungen und Bestimmung der Analyten PGI und G-17 sowie eines Markers für *Helicobacter pylori* in der Referenzpopulationsgruppe, wobei die auf diese Weise durch das Datenverarbeitungssystem erzeugten Daten den Zustand der Magenschleimhaut in dem Patienten anzeigen.

2. Verfahren nach Anspruch 1 zum Beurteilen einer Veränderung des Zustands der Magenschleimhaut, wobei das Verfahren Folgendes umfasst: Wiederholen der Bestimmung der Wahrscheinlichkeit der mindestens einen Magenschleimhautklasse und Vergleichen der auf diese Weise ermittelten Wahrscheinlichkeiten mit den früher bestimmten Wahrscheinlichkeiten, um Informationen über die Veränderung des Zustands der Magenschleimhaut bereitzustellen.

3. Verfahren nach einem der vorhergehenden Ansprüche, wobei die Wahrscheinlichkeiten bestimmt werden, indem ein statistisches Verfahren zur Berechnung der Klassifikationswahrscheinlichkeiten verwendet wird.

4. Verfahren nach Anspruch 3, wobei das statistische Verfahren zur Berechnung der Klassifikationswahrscheinlichkeiten ein multinominales logistisches Regressionsverfahren (MLR) ist.

5. Verfahren nach einem der vorhergehenden Ansprüche, umfassend den zusätzlichen Schritt der Verwendung der erzeugten Informationen, um eine Diagnose und/oder einen Vorschlag für weitere Behandlungen oder Untersuchungen zu liefern.

6. Verfahren nach einem der vorhergehenden Ansprüche, wobei der Marker für *Helicobacter pylori* ein *Helicobacter pylori*-Antikörper ist, dessen Konzentration in der Probe gemessen wird.

7. Verfahren nach einem der vorhergehenden Ansprüche, wobei der Marker für *Helicobacter pylori* ein *Helicobacter pylori*-Antigen ist, dessen Gegenwart in der Probe bestimmt wird.

8. Verfahren nach einem der vorhergehenden Ansprüche, wobei der gemessene Gastrinwert der stimulierte Gastrin-17-Wert (G-17st) oder sowohl der Gastrin-17-als auch der stimulierte Gastrin-17-Wert ist.

9. Verfahren nach einem der vorhergehenden Ansprüche, wobei zusätzlich die Konzentration des Analyten Pepsinogen II (PGII) gemessen wird und das PGI/PGII-Verhältnis bei der statistischen Berechnung verwendet wird.

10. Verfahren nach Anspruch 1, wobei die Analyten in einer Körperflüssigkeit gemessen werden, wie z.B. einer Serum-, Vollblut-, Urin-, Speichel- oder Tränenflüssigkeitsprobe, insbesondere einer Serumprobe.

11. Verfahren nach einem der vorhergehenden Ansprüche, wobei die Datenverarbeitungsmittel eine Anzeige umfassen und die erzeugten Informationen auf der Anzeige angezeigt werden.

12. Ausrüstung, umfassend Mittel zur Bestimmung der Konzentration von Pepsinogen I und Gastrin-17 und der Konzentration oder Gegenwart eines Markers für *Helicobacter pylori* in einer Probe sowie ein Computerprogrammprodukt, das auf einem computerlesbaren Medium enthalten ist und Computercodemittel umfasst, die geeignet sind, eine Wahrscheinlichkeit einer Magenschleimhautklasse zu bestimmen, wobei die Magenschleimhautklasse unter der Gruppe von Klassen gewählt wird, die aus Folgendem besteht: normal (N), Antrumatrophie (A), Antrum- und Corpusatrophie (AC), Corpusatrophie (C) und oberflächliche oder nichtatrophische Gastritis (S), und zwar auf der Basis von Messwerten für die Analyten und/oder Marker sowie auf der Basis vordefinierter klinischer Daten in einer Datenbank, wobei die vordefinierten klinischen Daten solche Daten umfassen, die in einer Referenzpopulationsgruppe gewonnen wurden, und zwar durch gastroskopische Untersuchungen und Bestimmung von Werten für die Analyten PGI und G-17 sowie eines Markers für *Helicobacter pylori* in der Referenzpopulationsgruppe, wobei die Mittel geeignet sind, als Reaktion auf diese Bestimmung und optional auf andere eingegebene Daten Informationen zu liefern, wenn sie in einem Computer ausgeführt werden.

13. Computerprogrammprodukt, das auf einem computerlesbaren Medium enthalten ist und Computercodemittel umfasst, die geeignet sind, eine Wahrscheinlichkeit einer Magenschleimhautklasse zu bestimmen, wobei die Magenschleimhautklasse unter der Gruppe von Klassen gewählt wird, die aus Folgendem besteht: normal (N), Antrumatrophie (A), Antrum- und Corpusatrophie (AC), Corpusatrophie (C) und oberflächliche oder nichtatrophische Gastritis (S), und zwar auf der Basis von Messwerten für die Analyten PGI und G-17 und für den *Helicobacter pylori*-Marker sowie auf der Basis vorbestimmter klinischer Daten in einer Datenbank, wobei die Mittel geeignet sind, als Reaktion auf diese Bestimmung und optional auf andere eingegebene Daten Informationen zu liefern, wenn sie in einem Computer ausgeführt werden.

**Revendications**

1. Procédé d'évaluation ou de prédiction de l'état de la muqueuse gastrique chez un sujet en déterminant, chez ledit sujet, la probabilité que la muqueuse gastrique appartienne à au moins une classe de muqueuse gastrique, ledit procédé comprenant les étapes consistant à

- mesurer, dans un échantillon dudit sujet, les concentrations de substances à analyser que sont le pepsinogène I (PGI) et la gastrine-17 (G-17), ainsi que déterminer la présence ou la concentration d'un marqueur d'*Helicobacter pylori,*
- entrer les données ainsi obtenues dans un système de traitement de données comprenant un système d'exploitation, une base de données et un moyen pour émettre et recevoir et traiter les données, ledit système de traitement de données étant adapté pour déterminer la probabilité de la muqueuse gastrique appartenant à au moins la classe de muqueuse gastrique, la classe de muqueuse gastrique étant choisie dans l'ensemble des classes constituées de la normale (N), une atrophie de l'antre (A), une atrophie de l'antre et du corps (AC), une atrophie du corps (C) et une gastrite superficielle ou non atrophique (S), sur la base des données entrées ainsi que de données cliniques prédéfinies dans la base de données, les données cliniques prédéfinies dans la base de données comprenant des données obtenues à partir d'un groupe de population de référence au moyen d'une étude gastroscopique et par détermination des substances à analyser PGI et G-17 et du marqueur d'*Helicobacter pylori* dans ledit groupe de la population de référence, les informations ainsi générées par le système de traitement de données étant indicatives de l'état de la muqueuse gastrique chez ledit sujet.

2. Procédé selon la revendication 1 pour évaluer un changement de l'état de la muqueuse gastrique, ledit procédé comprenant les étapes consistant à répéter la détermination de la probabilité de la classe de muqueuse gastrique, au nombre d'au moins une, et à comparer les probabilités ainsi obtenues aux probabilités déterminer auparavant de façon à fournir des informations concernant le changement de l'état de la muqueuse gastrique.

3. Procédé selon l'une quelconque des revendications précédentes, dans lequel on détermine les probabilités en utilisant une méthode statistique pour le calcul des probabilités de classification.

4. Procédé selon la revendication 3, dans lequel la méthode statistique pour le calcul des probabilités de classification est une méthode de régression logistique multinominale (MLR).

5. Procédé selon l'une quelconque des revendications précédentes, comprenant l'étape supplémentaire d'utilisation des informations générées pour fournir un diagnostic et/ou une suggestion pour des traitements ou des examens supplémentaires.

6. Procédé selon l'une quelconque des revendications précédentes, dans lequel le marqueur d'*Helicobacter pylori* est un anticorps dirigé contre *Helicobacter pylori* dont la concentration est mesurée à partir de l'échantillon.

7. Procédé selon l'une quelconque des revendications précédentes, dans lequel le marqueur d'*Helicobacter pylori* est l'antigène *Helicobacter pylori* dont la présence est déterminée dans l'échantillon.

8. Procédé selon l'une quelconque des revendications précédentes, dans lequel la valeur mesurée de gastrine est la valeur de gastrine-17 stimulée (G-17st) ou à la fois de la gastrine-17 et de la gastrine-17 stimulée.

9. Procédé selon l'une quelconque des revendications précédentes, dans lequel, de plus, on mesure la concentration de la substance à analyser pepsinogène II (PGII) et on utilise le rapport PGI/PGII dans le calcul statistique.

10. Procédé selon la revendication 1, dans lequel on mesure les substances à analyser à partir d'un fluide corporel comme un échantillon de sérum, de sang entier, d'urine, de salive ou de fluide lacrymal, en particulier un échantillon de sérum.

11. Procédé selon l'une quelconque des revendications précédentes, dans lequel le moyen de traitement de données comprend un afficheur, et les informations générées sont affichées sur l'afficheur.

12. Kit comprenant un moyen pour déterminer, à partir d'un échantillon, la concentration de pepsinogène I et de gastrine-17, et la concentration ou la présence d'un marqueur d'*Helicobacter pylori,* ainsi qu'un produit de programme informatique réalisé sur un support pouvant être lu avec un ordinateur et comprenant un moyen de code informatique adapté pour déterminer une probabilité d'une classe de muqueuse gastrique, la classe de muqueuse gastrique étant choisie dans l'ensemble des classes constituées de la normale (N), une atrophie de l'antre (A), une atrophie de l'antre et du corps (AC), une atrophie du corps (C) et une gastrite superficielle ou non atrophique (S), sur la base de valeurs mesurées pour lesdites substances à analyser et/ou le marqueur, ainsi que de données cliniques prédéfinies dans une base de données, les données cliniques prédéfinies comprenant des données obtenues à partir d'un groupe de population de référence au moyen d'études gastroscopiques et par détermination des valeurs des substances à analyser PGI et G-17 et du marqueur d'*Helicobacter pylori* dans ledit groupe de la population de référence, et pour fournir des informations en réponse à ladite détermination et éventuellement à d'autres données entrées, lors d'une exécution dans un ordinateur.

13. Produit de programme informatique réalisé sur un support pouvant être lu avec un ordinateur et comprenant un moyen de code informatique adapté pour déterminer une probabilité d'une classe de muqueuse gastrique, la classe de muqueuse gastrique étant choisie dans l'ensemble des classes constituées de la normale (N), une atrophie de l'antre (A), une atrophie de l'antre et du corps (AC), une atrophie du corps (C) et une gastrite superficielle ou non atrophique (S), sur la base de valeurs mesurées pour les substances à analyser et/ou le marqueur d'*Helicobacter pylori,* ainsi que de données cliniques prédéfinies dans une base de données, et pour fournir des informations en réponse à ladite détermination et éventuellement à d'autres données entrées, lors d'une exécution dans un ordinateur.

GastroPanel trend: time series of the probabilities for different classes (A, AC, C, S, N) provides valuable tool for follow up after treatment (eg. eradication therapy).

Fig. 1

GastroMap – graphical presentation of atrophic classes (A, AC, C, S, N) as a function of HPAB (EIU) and PGI (ug/l), in case on G-17 = 3 pmol/l.

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9615456 A
- WO 0067035 A
- WO 02054084 A
- WO 2004023148 A

**Non-patent literature cited in the description**

- **SIPPONEN, P ; KEKKI, M ; HAAPAKOSKI, J. ; IHAMÄKI, T ; SIURALA, M.** Gastric cancer risk in chronic atrophic gastritis: statistical calculations of cross-sectional data. *Int J Cancer,* 1985, vol. 35, 173-77
- **CALAM, J.** Helicobacter pylori (Review). *Eur. J. Clin Invest,* 1994, vol. 24, 501-510